# FASCICULE DE BREVET EUROPEEN

(11) **EP 1 351 917 B1**
(45) Date de publication et mention de la délivrance du brevet: **06.04.2005**
(21) Numéro de dépôt: 02711994.0
(22) Date de dépôt: 17.01.2002
(51) Int. Cl.: C07C 65/00, C07C 51/42, C07C 63/24, C07C 63/26, C07C 63/04, C07C 53/126

(54) **COMPOSITIONS STABILISEES D'ACIDE O-IODOXYBENZOIQUE ET LEUR PROCEDE DE PREPARATION**
STABILISIERTE O-IODOXYBENZOESÄUREZUSAMMENSETZUNGEN UND VERFAHREN ZU IHRER HERSTELLUNG
STABILISED O-IODOXYBENZOIC ACID COMPOSITIONS AND PREPARATION METHOD

(30) Priorité: 19.01.2001 FR 0100759
(43) Date de publication de la demande: 15.10.2003
(73) Titulaire: Simafex, 17230 Marans (FR)
(72) Inventeur: DEPERNET, Dominique, F-17000 La Rochelle (FR); FRANCOIS, Bruno, F-17410 Saint Martin de Ré (FR)
(74) Mandataire: Bernasconi, Jean Raymond
(86) Numéro de dépôt international: PCT/FR2002/000189
(87) Numéro de publication internationale: WO 2002/057210

(56) Documents cités:
- US-A- 2 566 592

## Description

L'invention concerne des compositions stabilisées d'acide o-iodoxybenzoïque, ou IBX, de formule 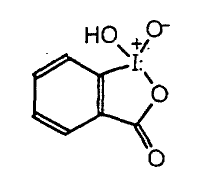 et leur procédé de préparation.

Ce dérivé d'iode hypervalent est le précurseur du 1,1,1-triacetoxy-1,1-dihydro-1,2-benzodioxol-3(1H)-one ou periodinane de Dess-Martin, de formule 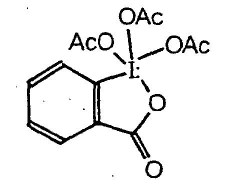 décrit pour la première fois dans J. Org. Chem. 1983, 48, 4155-4156 comme oxydant doux et sélectif d'alcools primaires et secondaires en composés carbonylés.

Malgré cette importante propriété, les risques encourus lors de la manipulation de ce réactif et celle de son précurseur, l'IBX, entravent leur mise dans le commerce et même leur utilisation en synthèse chimique, au-delà du stade du laboratoire.

Ces 2 composés se décomposent violemment comme mentionné par J.B. Plumb et D.J. Harper dans Chemical and Engineering News p3 16 Juillet 1990 ou dans J. Org. Chem. 1993, 58, 2899, article dans lequel un procédé amélioré de préparation du periodinane de Dess-Martin à partir de l'IBX est décrit.

L'IBX peut être préparé par oxydation de l'acide 2-iodobenzoïque notamment par KBrO₃ dans l'acide sulfurique, procédé classique plusieurs fois perfectionné, ou par l'Oxone®, constituée de 2KHSO₅.KHSO₄.K₂SO₄, comme décrit dans J. Org. Chem. 1999, 64, 4537-4538. Les auteurs de cet article récent confirment que l'IBX obtenu est un solide cristallin, blanc, qui explose même très pur.

La demanderesse a elle-même vérifié que le periodinane de Dess-Martin se décompose violemment à sa température de fusion et que l'IBX, même humidifié avec de l'eau ou de l'acide acétique, présente des propriétés explosives, quand il est soumis à l'action de la chaleur selon la méthode A14 de la Directive 92/69/CEE.

Il était donc souhaitable de trouver un moyen de stabiliser l'acide o-iodoxybenzoïque dès sa préparation afin de permettre son utilisation en .synthèse organique sans risques majeurs.

Le sorbitol, proposé il y a 50 ans dans US 2,566,592 comme agent stabilisant de l'IBX et de ses sels de calcium et d'ammonium pour permettre leur utilisation en thérapeutique, ne paraît pas réellement efficace et surtout ne peut convenir lorsque l'IBX doit être mis en oeuvre dans une réaction d'oxydation d'alcools.

La demanderesse a maintenant trouvé d'autres agents stabilisants de l'IBX, qui n'entravent pas son utilisation comme agent oxydant, et la composition constituée d'une quantité convenable d'au moins un de ces agents et d'IBX n'explose plus sous l'action d'un choc ou d'une élévation importante de température de telle sorte que ce periodinane pourra être utilisé plus largement en synthèse, particulièrement à la place du réactif de Dess-Martin.

M. Frigerio, M. Santogostino, S. Sputore et G. Palmisano ont rapporté dans J. Org. Chem. 1995, 60, 7272-7276 que l'IBX dont l'insolubilité dans de nombreux solvants organiques et dans l'eau limitait l'utilisation, avait en solution dans le diméthylsulfoxyde des propriétés oxydantes comparables à celles du réactif de Dess-Martin, sans présenter la sensibilité à l'humidité de ce dernier, rapportée notamment dans J. Org.Chem. 1994, 59, 7549-7552.

A titre d'exemple de cette utilisation, on peut citer outre les réactions décrites par M. Frigerio et coll., celles résultant des travaux de K.C. Nicolaou et coll., décrits notamment dans Angew. Chemie, Int. Ed. 2000, 39, 625-628 et 2525-2529 ou pour des anilides dans Angew. Chemie 2000, 112(3), 639-642.

On a maintenant constaté que ces réactions pouvaient être effectuées dans d'autres solvants aprotiques polaires que le diméthylsulfoxyde avec les compositions stabilisées de l'invention, notamment dans le tetrahydrofuranne ou la N-méthyl pyrrolidone.

La présente invention concerne les compositions stabilisées d'acide o-iodoxybenzoïque qui contiennent pour 1 mole d'acide o-iodoxybenzoïque de 0,5 à 4 moles d'un agent stabilisant choisi parmi
- les acides aliphatiques de formule I

   CH₃(CH₂)ₙ COOH

   dans laquelle n est compris entre 8 et 20 et de préférence égal à 14 ou 16, ou leurs mélanges,
   - les acides benzènecarboxyliques de formule II 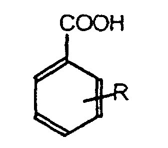 dans laquelle R représente H, CH₃, COOH
      ou leurs mélanges
   - ainsi que les mélanges d'acides de formule I et II.

On préfère les compositions dans lesquelles, il y a de 1 à 2,5 moles d'agent stabilisant par mole d'acide o-iodoxybenzoïque, et mieux de 1,8 à 2,2 moles, afin d'assurer une stabilisation maximale sans augmenter exagérément les coûts.

Parmi les acides de formule II, on préfère les acides benzoïque, toluiques, isophtalique et téréphtalique et parmi eux les acides de points de fusion supérieurs à 200°C comme l'acide isophtalique et l'acide téréphtalique, température généralement admise comme étant celle à laquelle l'IBX explose spontanément.

Néanmoins, les mélanges d'acide benzoïque ou d'acide o-toluique ou d'un de leurs mélanges d'une part et d'acide isophtalique ou d'acide téréphtalique ou d'un de leurs mélanges d'autre part, à raison de 25 à 75% en mole du monoacide dans lé mélange et mieux de 45 à 55% donnent des compositions stabilisées qui n'explosent pas sous l'action d'un choc ou de la chaleur.

On préfère les compositions d'acide iodoxybenzoïque contenant à titre d'agent stabilisant un ou plusieurs composés de formule II.

Il est préférable dans les compositions à base d'agent stabilisant de formule I d'introduire aussi un agent de formule II et on préfère les mélanges d'une part d'acide stéarique ou palmitique ou d'un de leurs mélanges, acides largement répandus, et d'autre part d'acide téréphtalique ou d'acide isophtalique ou d'un de leurs mélanges, à raison de 25 à 75% en mole d'acide gras dans le mélange.

L'invention concerne aussi le procédé de préparation des compositions de l'invention soit à partir d'IBX déjà isolé soit lors de la synthèse de l'IBX.

Pour préparer une composition à partir d'IBX déjà isolé ou pour homogénéiser une composition de l'invention, on réalise une solution ou suspension dans l'eau des sels alcalins, particulièrement de sodium, des acides constituant la composition puis on acidifie jusqu'à insolubilisation totale de la composition.

On peut soit ajouter une solution d'un hydroxyde alcalin à la suspension des acides constituants dans l'eau soit ajouter lesdits acides à une solution aqueuse d'hydroxyde alcalin en quantité suffisante.

On peut aussi introduire une quantité convenable de l'un des agents stabilisants de formule I ou II, l'un de ses sels alcalins ou l'un de leurs mélanges dans le milieu dans lequel l'IBX est préparé, de préférence en totalité ou en partie dès le début de la réaction, si l'agent stabilisant n'est pas décomposé dans ce milieu réactionnel. Lorsque l'agent stabilisant est un mélange, l'un des constituants pourra être ajouté en cours de réaction et l'autre avant l'isolement de l'IBX.

L'homme du métier pourra lors de quelques essais préalables déterminer les proportions relatives du ou des agents stabilisants et de l'IBX ainsi que les modes d'introduction de ces agents, en prenant en compte leurs éventuelles réactivités et leurs solubilités relatives dans les milieux réactionnels et de précipitation.

Dans ce qui suit, on décrit des exemples de l'invention et de leur application comme agents oxydants.

La concentration d'IBX dans les compositions obtenues a été déterminée par dosage avec du thiosulfate de sodium, de l'iode libérée lors de l'oxydation de KI par l'IBX présent dans la composition (essai oxydimétrique ).

Les essais d'explosivité ont été effectués au laboratoire :
- soit en soumettant au choc d'un marteau, 100 mg d'une composition
- soit en projetant sur une plaque chauffée à 300°C quelques mg de composition ou en chauffant une plaque sur laquelle quelques mg ont été déposés, jusqu'à décomposition.

### Exemple 1

a) Préparation d'IBX
   On met en suspension 300g d'acide 2-iodobenzoïque dans 3 I d'eau contenant 201g de H₂SO₄ à 96%.Puis, sous agitation à 50°C, on introduit en 30 minutes, 246g de NaBrO₃ dans 1,2 I d'eau. Le milieu est alors maintenu à 65°C pendant 3 heures 30, au cours desquelles se produit un fort dégagement de brome. Après refroidissement à 20°C, l'IBX précipité dans le milieu est isolé avec précaution, lavé avec 1l d'eau et séché sous vide.
   Rendement : 95%
   100mg de cette poudre soumis à un choc donne un fort dégagement de fumée; sur une plaque chauffante, la poudre explose avec un flash orangé et un fort dégagement de fumée.
b) 10g d'IBX sont mis en suspension dans 60ml d'eau et 2,2g d'acide benzoïque ( 0,5 équivalent molaire ) sont ajoutés avant l'introduction lente. sous agitation d'environ 6ml de solution aqueuse de NaOH 10N; jusqu'à dissolution complète. Le milieu est alors acidifié lentement par addition de 6 ml d'une solution aqueuse d'HCl à 33%. Le précipité formé est isolé par filtration, lavé avec 30ml d'eau et séché à 60°C, sous vide.
   Rendement pondéral : 90%
   On obtient une composition qui par chauffage donne une épaisse fumée grise mais pas de flamme orangée.

### Exemple 2

On introduit 30g d'acide 2-iodobenzoïque dans une solution de 109g d'Oxone dans 390ml d'eau. Le milieu réactionnel hétérogène est maintenu sous agitation à 70°C pendant 2 heures au cours desquelles il épaissit. On ajoute alors 18g d'acide benzoïque et on laisse refroidir le mélange jusqu'à température ambiante, 20°C environ. Le précipité est isolé, lavé avec 100ml d'eau et séché sous vide à 60°C.

Rendement : 90%

La composition obtenue qui contient 1,2 mole de stabilisant par mole d'IBX est stable au choc et au chauffage dans les essais habituels; elle se décompose en produisant une fumée grise, mais pas de flamme orangée, lorsqu'on pratique l'essai à la chaleur sur 500mg.

### Exemple 3

On introduit 30g d'acide 2-iodobenzoïque dans une solution de 92g d'Oxone dans 300ml d'eau. Après 2 heures à 70°C on ajoute 18g d'acide o-toluique et filtre le milieu revenu à 20°C.Après lavage du solide avec 100ml d'eau et séchage, on obtient une poudre qui est décomposée par la chaleur sans explosion.

### Exemple 4

On ajoute 50g d'acide 2-iodobenzoïque et 37g d'acide isophtalique dans une solution de 153g d'Oxone dans 500ml d'eau. Le milieu hétérogène est maintenu 2 heures à 70°C puis il est refroidi jusqu'à température ambiante avant filtration. Le précipité est lavé à l'eau puis mis en suspension dans 800ml d'eau avant l'addition de 60ml environ d'une solution aqueuse de NaOH 10N, pour dissoudre les acides. Une composition selon l'invention homogénéisée est ensuite précipitée par acidification du milieu, par addition de 60ml d'une solution aqueuse de HCl à 33%. Après lavage à l'eau et séchage, on obtient 80g de composition stable, qui n'explose pas par élévation de température mais fond en noircissant.

### Exemple 5

On ajoute 10g d'acide stéarique dans une suspension de 10g d'IBX dans 60ml d'eau avant d'introduire goutte à goutte, 8ml de solution aqueuse de NaOH 10N pour salifier les acides. Le milieu hétérogène est alors acidifié par addition de 8 ml d'une solution aqueuse de HCl à 33% avant l'isolement de la phase solide par filtration. La composition obtenue après lavage avec 30ml d'eau et séchage n'explose pas à la chaleur.

### Exemple 6

On introduit 32g d'acide 2-iodobenzoïque et 32g d'acide stéarique dans une solution de 117g d'Oxone et 490ml d'eau. Après 2 heures à 70°C le milieu est traité comme précédemment; la composition isolée généralement n'explose pas à la chaleur.

### Exemple 7

On introduit 280g d'acide2-iodobenzoïque, 187g d'acide isophtalique et 151g d'acide benzoïque dans 1014g d'Oxone dans 4600ml d'eau. Après 2 heures à 70°C et traitement habituel, on isole 605g de composition stable à la chaleur qui contient 46% en poids d'IBX, déterminé par oxydimétrie.

### Exemple 8

On utilise les même proportions de réactifs que dans l'exemple précédent mais on n'introduit l'acide benzoïque et l'acide isophtalique qu'après l'oxydation au cours du refroidissement du milieu, vers 35°C. La composition isolée par filtration est ensuite homogénéisée. Elle est mise en suspension dans 4100ml d'eau et 440ml de solution aqueuse de NaOH 10N sont ajoutés, la solution obtenue de pH 7 est ensuite acidifiée à 78°C par addition lente de 440ml d'une solution aqueuse d'acide chlorhydrique à 33%.La composition isolée après filtration, lavage avec 500ml d'eau et séchage sous vide contient 45% en poids d'IBX (oxydimétrie ). Elle est stable au choc et n'explose pas par chauffage.

### Exemple 9

On opère comme dans l'exemple 8 mais en remplaçant l'acide benzoïque par un équivalent molaire d'acide stéarique. On obtient ainsi une composition non explosive qui contient 38% en poids d'IBX.

### Exemple 10

On introduit dans une solution de 695 g d'Oxone dans 2,8 litres d'eau, 200 g d'acide o-iodobenzoïque et 133 g d'acide isophtalique. Le milieu est maintenu 3 heures sous agitation à 70°C puis on y ajoute à 40°C une solution de 128 g de benzoate de sodium dans 500 ml d'eau. Après refroidissement à 20°C, on filtre le précipité, le lave avec 700 ml d'eau et le sèche en étuve ventilée à 60°C pour obtenir 420 g de composition d'IBX stabilisée.

### Exemple d'application n° 1

Dans une solution de 1,6g de farnésol dans 40ml de diméthylsulfoxyde, on introduit en 15 minutes par petites fractions 5g de la composition préparée à l'exemple 7, en maintenant la température entre 20°C et 25°C. Après 2 heures sous agitation, la solution est versée dans 100ml d'hexanes ( essence G ) et on sépare le précipité qui est lavé à l'eau puis à l'essence G. Les phases organiques sont concentrées sous vide et le résidu obtenu est purifié par chromatographie sur 25g de silice R60 Merck® pour séparer le reste d'agent stabilisant du farnesal obtenu; l'élution est réalisée avec l'essence G puis son mélange avec l'acétate d'éthyle ( 9/1-v/v ). Le famésal pur est isolé avec 85% de rendement.

### Exemple d'application n° 2

a) Dans le diméthylsulfoxyde
   On ajoute par petites fractions 16g de la composition préparée comme à l'exemple 8 en 15 minutes, en maintenant la température entre 20°C et 25°C, dans 35ml de diméthylsulfoxyde contenant 4,8g de benzoïne. Après 2 heures d'agitation à température ambiante, le milieu est versé sur 200ml d'eau et 60ml d'acétate d'éthyle.Le précipité formé est éliminé et la phase organique concentrée sous vide. Le résidu solide est recristallisé dans 20ml d'éthanol. On obtient le benzile avec 78% de rendement.
b) Dans le tétrahydrofurane
   On ajoute par petites fractions 8,35 g de composition stabilisée préparée comme à l'exemple 10 dans une solution de 2,5 g de benzoïne dans 40 ml de tétrahydrofuranne à 20°C, puis on maintient le mélange sous agitation à 50°C pendant 6 heures. A 20°C, le milieu est introduit en agitant dans 100 ml d'eau et 60 ml de toluène ; on neutralise alors la phase aqueuse par addition d'une solution de NaOH 5N, filtre l'insoluble et sépare la phase organique. Après lavage par 60 ml d'eau et évaporation du solvant, on obtient 2 g de la dicétone attendue (spectre RMN conforme).
c) Dans la N-méthylpyrrolidone
   On opère comme dans le tétrahydrofuranne pour obtenir après traitement 2,6 g du produit attendu.

### Exemple d'application n°3

On ajoute par petites fractions en maintenant la température vers 20°C, en 15 minutes, 30,6 g de la composition de l'exemple 10 à une solution de 4,7 g de 3-pyridylméthanol dans 70 ml de diméthylsufoxyde et on maintient le milieu sous agitation pendant 2 heures avant de le verser sur 150 ml d'eau et 60 ml d'acétate d'éthyle. Après neutralisation de la phase aqueuse et séparation du précipité, la phase organique est séparée et amenée à sec. On obtient 4,2 g de la 3-pyridyl carboxaldéhyde attendue (RMN conforme).

### Exemple d'application n° 4

On met en suspension 10,6 g d'IBX stabilisé préparé comme à l'exemple 10 dans 60 ml de N-méthylpyrrolidone à 20°C et on ajoute, goutte à goutte, 2 g de cyclooctanol en maintenant la température entre 20°C et 25°C. Après 2 heures d'agitation, on verse le milieu dans un mélange de 130 ml d'eau et 80 ml de toluène ; le pH de la phase aqueuse est amené vers 7 par addition de NaOH aqueux et la phase organique après séparation de l'insoluble formé et de la phase aqueuse est concentrée pour donner 1,4 g de cyclooctanone pure (RMN et CPG).

### Exemple d'application n° 5

En appliquant le même mode opératoire que précédemment on obtient avec 87 % de rendement la menthone à partir de L-menthol.

### Exemple d'application n° 6

En appliquant le mode opératoire de l'exemple d'application n° 4, on obtient avec 86% de rendement la 3,4,5-triméthoxybenzaldéhyde à partir de l'alcool 3,4,5-trimethoxy benzylique ou l'adamantanone avec 52 % de rendement à partir d'adamantanol.

## Revendications

1. Composition stabilisée d'acide o-iodoxybenzoïque qui contient pour 1 mole d'acide o-iodoxybenzoïque de 0,5 à 4 moles d'un agent stabilisant choisi parmi les acides aliphatiques de formule I
CH₃(CH₂)ₙCOOH
dans laquelle n est compris entre 8 et 20 et les acides benzènecarboxyliques de formule II 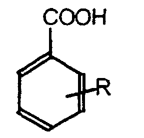 dans laquelle R représente H, CH₃, COOH
ou leurs mélanges.

2. Composition selon la revendication 1 dans laquelle l'agent stabilisant est un acide de formule I dans laquelle n est égal à 14 ou 16 ou un de leurs mélanges.

3. Composition selon la revendication 1 dans laquelle l'agent stabilisant est un acide de formule II ou un de leurs mélanges.

4. Composition selon la revendication 3 dans laquelle l'agent stabilisant est l'acide isophtalique, l'acide téréphtalique ou leurs mélanges.

5. Composition selon la revendication 1 dans laquelle l'agent stabilisant est un mélange d'acide benzoïque ou d'acide o-toluique ou d'un de leurs mélanges d'une part et d'acide isophtalique, d'acide téréphtalique ou d'un de leurs mélanges d'autre part, à raison de 25 à 75 % en mole de monoacide dans l'agent stabilisant.

6. Composition selon la revendication 5 dans laquelle l'agent stabilisant est un mélange d'acide benzoïque et d'acide isophtalique, à raison de 45% à 55% en mole de monoacide dans l'agent stabilisant.

7. Composition selon la revendication 1 dans laquelle l'agent stabilisant est un mélange d'acide stéarique, d'acide palmitique ou d'un de leurs mélanges d'une part et d'acide isophtalique, d'acide téréphtalique ou d'un de leurs mélanges d'autre part, à raison de 25% à 75% en mole de monoacide dans l'agent stabilisant.

8. Composition selon l'une quelconque des revendications précédentes qui contient de 1 à 2,5 moles d'agent stabilisant par mole d'acide o-iodoxybenzoïque.

9. Procédé de préparation d'une composition selon l'une des revendications 1 à 8 qui consiste à faire une solution ou une suspension aqueuse des sels alcalins de l'acide o-iodoxybenzoïque et de l'agent stabilisant en proportions convenables, puis à acidifier avant d'isoler la composition précipitée.

10. Procédé de préparation d'une composition stabilisée d'acide o-iodoxybenzoïque selon l'une des revendications 1 à 8 qui consiste à introduire l'agent stabilisant ou l'un de ses sels alcalins dans le milieu réactionnel de synthèse de l'acide o-iodoxybenzoïque.

11. Procédé selon l'une des revendications 9 ou 10 dans lequel l'acide o-iodoxybenzoïque est préparé par action de 2KHSO₅,KHSO₄,K₂SO₄ sur l'acide o-iodobenzoïque.

12. Procédé selon la revendication 11 dans lequel on introduit l'agent stabilisant en totalité ou en partie dès le début de la synthèse.

13. Procédé selon l'une des revendications 9 ou 10 dans lequel l'acide o-iodoxybenzoïque est préparé par action de NaBrO₃ et H₂SO₄ sur l'acide o-iodobenzoïque.

## Patentansprüche

1. Stabilisierte o-Jodoxybenzosäurezusammensetzung, enthaltend auf ein Mol o-Jodoxybenzoesäure 0,5 bis 4 Mol eines Stabilisierungsmittels, ausgewählt aus den aliphatischen Säuren der Formel I
CH₃(CH₂)ₙCOOH,
worin n eine Zahl zwischen 8 und 20 ist und den Benzolcarbonsäuren der Formel II 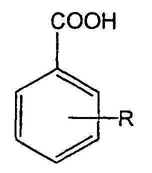 worin R gleich H, CH₃, COOH ist, oder deren Gemische.

2. Zusammensetzung nach Anspruch 1, in der das Stabilisierungsmittel eine Säure der Formel I ist, worin n gleich 14 oder 16 ist, oder ein Gemisch hiervon.

3. Zusammensetzung nach Anspruch 1, in der das Stabilisierungsmittel eine Säure der Formel II ist oder ein Gemisch hiervon.

4. Zusammensetzung nach Anspruch 3, in der das Stabilisierungsmittel Isophthalsäure, Terephthalsäure oder deren Gemische ist.

5. Zusammensetzung nach Anspruch 1, in der das Stabilisierungsmittel ein Gemisch aus Benzoesäure oder o-Methylbenzoesäure oder aus einem Gemisch hiervon einerseits, und Isophthalsäure, Terephthalsäure oder einem Gemisch hiervon andererseits in einer Menge von 25 bis 75 Mol%, bezogen auf die einwertige Säure in dem Stabilisierungsmittel, ist.

6. Zusammensetzung nach Anspruch 5, in der das Stabilisierungsmittel ein Gemisch aus Benzoesäure und Isophthalsäure in einer Menge von 45 bis 55 Mol%, bezogen auf die einwertige Säure in dem Stabilisierungsmittel, ist.

7. Zusammensetzung nach Anspruch 1, in der das Stabilisierungsmittel ein Gemisch aus Stearinsäure, Palmitinsäure oder aus einem Gemisch hiervon einerseits und Isophthalsäure, Terephthalsäure oder einem Gemisch hiervon andererseits in einer Menge von 25 bis 75 Mol%, bezogen auf die einwertige Säure in dem Stabilisierungsmittel, ist.

8. Zusammensetzung nach einem der vorangehenden Ansprüche, enthaltend 1 bis 2,5 Mol Stabilisierungsmittel pro Mol o-Jodoxybenzoesäure.

9. Verfahren zur Herstellung einer Zusammensetzung nach einem der Ansprüche 1 bis 8, bestehend aus dem Herstellen einer Lösung oder einer wässrigen Suspension von Alkalisalzen der o-Jodoxybenzosäure und dem Stabilisierungsmittel in zweckmäßigen Verhältnissen und anschließendem Ansäuern vor dem Isolieren der ausgefällten Zusammensetzung.

10. Verfahren zur Herstellung einer stabilisierten o-Jodoxybenzoesäure nach einem der Ansprüche 1 bis 8, bestehend aus dem Einbringen des Stabilisierungsmittels oder eines seiner Alkalisalze in das Reaktionsmedium der Herstellung von o-Jodoxybenzoesäure.

11. Verfahren nach einem der Ansprüche 9 oder 10, in dem die o-Jodoxybenzoesäure durch das Einwirken von 2KHSO₅, KHSO₄, K₂SO₄ auf o-Jodbenzoesäure hergestellt wird.

12. Verfahren nach Anspruch 11, in dem das Stabilisierungsmittel auf einmal oder zum Teil mit Beginn der Synthese eingebracht wird.

13. Verfahren nach einem der Ansprüche 9 oder 10, in dem die o-Jodoxybenzoesäure durch das Einwirken von NaBrO₃ und H₂SO₄ auf o-Jodbenzoesäure hergestellt wird.

## Claims

1. Stabilised o-iodoxybenzoic acid composition which contains, per 1 mole of o-iodoxybenzoic acid, 0.5 to 4 moles of a stabiliser selected from among the aliphatic acids of formula I
CH₃(CH₂)ₙCOOH
wherein n is between 8 and 20
and the benzenecarboxylic acids of formula II 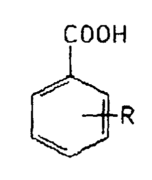 wherein R denotes H, CH₃, COOH
or mixtures thereof.

2. Composition according to claim 1, wherein the stabiliser is an acid of formula I where n is equal to 14 or 16 or one of the mixtures thereof.

3. Composition according to claim 1, wherein the stabiliser is an acid of formula II or one of the mixtures thereof.

4. Composition according to claim 3, wherein the stabiliser is isophthalic acid, terephthalic acid or mixtures thereof.

5. Composition according to claim 1, wherein the stabiliser is a mixture of benzoic acid or o-toluic acid or one of the mixtures thereof, on the one hand, and isophthalic acid, terephthalic acid or one of the mixtures thereof, on the other hand, in an amount of 25 to 75 mol-% of monoacid in the stabiliser.

6. Composition according to claim 5, wherein the stabiliser is a mixture of benzoic acid and isophthalic acid, in an amount of 45 to 55 mol-% of monoacid in the stabiliser.

7. Composition according to claim 1, wherein the stabiliser is a mixture of stearic acid, palmitic acid or one of the mixtures thereof, on the one hand, and isophthalic acid, terephthalic acid or one of the mixtures thereof, on the other hand, in an amount of 25 to 75 mol-% of monoacid in the stabiliser.

8. Composition according to any one of the preceding claims, which contains from 1 to 2.5 moles of stabiliser per mole of o-iodoxybenzoic acid.

9. Process for preparing a composition according to one of claims 1 to 8, which comprises making a solution or an aqueous suspension of the alkali metal salts of o-iodoxybenzoic acid and the stabiliser in suitable proportions, then acidifying it before isolating the composition precipitated.

10. Process for preparing a stabilised composition of o-iodoxybenzoic acid according to one of claims 1 to 8, which comprises adding the stabiliser or one of the alkali metal salts thereof to the reaction medium for synthesising the o-iodoxybenzoic acid.

11. Process according to one of claims 9 or 10, wherein the o-iodoxybenzoic acid is prepared by reacting 2KHSO₅, KHSO₄, K₂SO₄ with o-iodobenzoic acid.

12. Process according to claim 11, wherein all or some of the stabiliser is added right at the start of the synthesis.

13. Process according to one of claims 9 or 10, wherein the o-iodoxybenzoic acid is prepared by reacting NaBrO₃ and H₂SO₄ with o-iodobenzoic acid
